# EUROPÄISCHE PATENTSCHRIFT

(11) **EP 2 086 610 B1**
(45) Veröffentlichungstag und Bekanntmachung des Hinweises auf die Patenterteilung: **20.10.2010**
(21) Anmeldenummer: 07819634.2
(22) Anmeldetag: 07.11.2007
(51) Int. Cl.: A61M 5/14, A61J 1/10

(54) **TROPFKAMMER FÜR EIN INFUSIONSGERÄT**
DRIP CHAMBER FOR AN INFUSION DEVICE
CHAMBRE DE GOUTTE-À-GOUTTE POUR UN APPAREIL DE PERFUSION

(30) Priorität: 11.11.2006 DE 102006053219
(43) Veröffentlichungstag der Anmeldung: 12.08.2009
(73) Patentinhaber: Fresenius Kabi Deutschland GmbH, 61352 Bad Homburg v.d.H. (DE)
(72) Erfinder: RAHIMY, Ismael, 61169 Friedberg (DE); BRANDENBURGER, Torsten, Dipl.-Ing., 61203 Reichelsheim (DE)
(74) Vertreter: Luderschmidt, Schüler & Partner
(86) Internationale Anmeldenummer: PCT/EP2007/009618
(87) Internationale Veröffentlichungsnummer: WO 2008/058656

(56) Entgegenhaltungen:
- WO-A-2005/037362
- DE-A1- 19 748 497
- DE-U1- 20 216 791
- US-A1- 2005 124 942

## Beschreibung

Die Erfindung bezieht sich auf eine Tropfkammer für ein Infusionsgerät, mit dem medizinische Lösungen, beispielsweise Infusionslösungen für die Chemotherapie, einem Patienten zugeführt werden. Darüber hinaus betrifft die Erfindung ein Infusionsgerät mit einer Tropfkammer sowie eine Anordnung bestehend aus einem Infusionsbehältnis zur Aufnahme einer medizinischen Flüssigkeit und einem derartigen Infusionsgerät.

Die bekannten Infusionsgeräte verfügen über Tropfkammer, die ein Eintreten von Luft in die Infusionsleitung insbesondere beim Leerlaufen des Infusionsbehältnisses wirkungsvoll verhindern.

Die DE 197 48 497 A1 beschreibt ein Infusionsgerät mit einer Tropfkammer und einer Infusionsleitung. Die Tropfkammer weist einen im Wesentlichen zylindrischer Gehäusekörper mit einem distalen Einlass und einem proximalen Auslass auf. An dem distalen Einlass des Gehäusekörpers ist ein Anschlussteil zum Anschluss der Tropfkammer an einen Anschlussteil eines Infusionsbehältnisses vorgesehen. Der Anschlussteil der Tropfkammer weist einen Einstechdorn mit einem Flüssigkeitskanal auf, der zum Anschluss der Tropfkammer in einen Einstechteil des Anschlussteils des Infusionsbehältnisses eingestochen wird. An den proximalen Auslass des Gehäusekörpers ist eine Infusionsleitung angeschlossen, die mit einer Rollenklemme abgeklemmt werden kann. Die Infusionsleitung weist ein proximales Anschlussstück zum Anschluss einer Infusionskanüle auf.

Die bekannten Infusionsgeräte, die über einen Einstechdorn verfügen, haben den Vorteil, dass eine schnelle Verbindung zwischen dem Infusionsbehältnis, beispielsweise einem Infusionsbeutel, möglich ist. Nachteilig ist jedoch, dass die Gefahr des Lösens der Tropfkammer von dem Infusionsbeutel besteht. Insbesondere bei ruckartigen Bewegungen des Patienten, beispielsweise beim Essen, Trinken oder Gehen, kann sich der Einstechdorn der Tropfkammer leicht aus dem Einstechteil des Infusionsbehältnisses lösen, so dass das gesamte System nicht mehr dicht ist. Die Folge ist ein Auslaufen der Infusionslösung aus dem Infusionsbehältnis. Insbesondere bei der Anwendung von hochtoxischen Medikamenten, beispielsweise Zytostatika, besteht die Gefahr der Kontamination des Pflegepersonals oder der Besucher des Patienten.

Die US-A-5 735 826 beschreibt eine Anordnung bestehend aus einem Infusionsbehältnis, insbesondere einem Infusionsbeutel, und einem Infusionsgerät, das über eine Tropfkammer verfügt. Infusionsbeutel und Tropfkammer weisen jeweils Anschlussstücke auf, die eine nadellose Konnektion ermöglichen. Dadurch wird eine lösbare Verbindung ohne die Gefahr geschaffen, dass sich die Tropfkammer bei der Infusion von dem Infusionsbeutel löst. Nachteilig ist jedoch, dass das Infusionssystem spezielle Anschlussstücke vorsieht, die nicht über die in der Praxis bewährten Einstechdome und Einstechteile verfügen.

Aus der US-A-356 150 ist eine Tropfkammer für ein Infusionsgerät bekannt, die über einen Schraubverschluss verfügt, mit dem die Tropfkammer mit einem Infusionsbehältnis, insbesondere einer Infusionsflasche, verschraubt wird. Auch hier erweist sich als nachteilig, dass von den bewährten Einstechdomen und Einstechteilen nicht Gebrauch gemacht wird.

Die US 2005/0124942 A1 offenbart ein Infusionssystem, bei dem die Tropfkammer des Infusionsgerätes einen Einstechdorn aufweist, der in einen Einstechteil des Infusionsbehältnisses eingestochen wird. Zur Sicherung der Tropfkammer an dem Infusionsbehältnis dient eine Befestigungsklammer, die zwei einander gegenüberliegende Haken aufweist, die federnd an der Tropfkammer befestigt sind. Die beiden Haken hintergreifen den Anschlussteil des Infusiorisbehältnisses, wenn der Einstechdorn in den Einschlussteil eingestochen ist, so dass die Tropfkammer sicher an dem Infusionsbehältnis befestigt ist.

Das aus der US 2005/0124942 A1 bekannte Infusionssystem stellt zwar sicher, dass sich die Tropfkammer nicht unbeabsichtigt von dem Infusionsbehältnis lösen kann, nachteilig ist jedoch, dass noch seitliche Bewegungen der Tropfkammer in einem gewissen Spielraum möglich sind, wodurch der Einstechdorn in dem Einstechteil unerwünschten Beanspruchungen ausgesetzt ist. Im Übrigen ist die Handhabung beim Anschluss der Tropfkammer an das Infusionsbehältnis erschwert. Zum Anschluss der Tropfkammer an das Infusionsbehältnis müssen die beiden Haken der Befestigungsklammer radial nach außen gedrückt werden, um den Einstechdom in das Einstechteil des Behältnisses einstechen zu können.

Der Erfindung liegt die Aufgabe zu Grunde, eine einfach zu handhabende Tropfkammer für ein Infusionsgerät bereit zu stellen, die eine sichere Verbindung von Infusionsgerät und Infusionsbehältnis erlaubt. Darüber hinaus ist eine Aufgabe der Erfindung, ein einfach zu handhabendes Infusionsgerät mit einer derartigen Tropfkammer sowie eine einfach zu handhabende Anordnung bestehend aus einem Infusionsbehältnis und einer Tropfkammer zu schaffen, die eine sichere Verbindung ermöglichen.

Die Lösung dieser Aufgaben erfolgt erfindungsgemäß mit den Merkmalen der Patentansprüche 1, 8 und 9. Vorteilhafte Ausführungsformen der Erfindung sind Gegenstand der Unteransprüche.

Bei der erfindungsgemäßen Tropfkammer weist das Befestigungsteil zur Befestigung der Tropfkammer an dem Infusionsbehältnis eine Mehrzahl, d. h. mindestens drei Befestigungselemente auf, die entlang des Umfangs eines Kreises angeordnet sind. An den einander-zugewandten Innenseiten weisen die Befestigungselemente Nuten auf, die entlang des Umfangs eines Kreises verläufen.

Die Befestigungselemente sind derart ausgebildet, dass sie aus einer das Befestigungsteil des Anschlussteils des Infusionsbehältnisses fest umgreifenden Stellung in eine das Befestigungsteil des Infusionsgerätes freigebende Stellung spreizbar sind. Die Befestigungselemente der erfindungsgemäßen Tropfkammer umgreifen das Befestigungsteil des Infusionsbehältnisses von allen Seiten, so dass die Tropfkammer sicher an dem Infusionsbehältnis festgelegt ist. Dabei verhindern die umfangsmäßig angeordneten Befestigungselemente seitliche Bewegungen der Tropfkammer, so dass der Einstechdom in dem Einstechteil keinen unerwünschten Beanspruchungen ausgesetzt ist.

Während die Befestigungselemente des Befestigungsteils der Tropfkammer Nuten aufweisen, verfügt das Befestigungsteil des Anschlussteils des Infusionsbehältnisses einen umlaufenden Ansatz, der derart ausgebildet ist, dass der umlaufende Ansatz in den Nuten des Befestigungsteils der Tropfkammer einschnappend festgelegt ist. Dadurch wird erreicht, dass sich die Tropfkammer leicht an dem Infusionsbehältnis befestigen lässt. Für die einschnappende Befestigung der Tropfkammer des Infusionsbehältnis ist es grundsätzlich nicht erforderlich, die Befestigungselemente auseinander zu spreizen, da die Befestigungselemente beim Zusammenfügen der Anschlussteile von alleine nach außen gedrückt werden, bis der umlaufende Ansatz in die Nuten einschnappt.

Die Verbindung von Tropfkammer und Infusionsbehältnis kann eine grundsätzlich wieder lösbare Verbindung sein, auch wenn die Verbindung so ausgebildet ist, das sich Tropfkammer und Infusionsbehältnis bei dem Gebrauch der Anordnung nicht voneinander lösen lassen. Es ist aber auch möglich, die Verbindung so auszubilden, dass ein nachträgliches Lösen der Verbindung ohne Beschädigungen der Anschlussteile nicht möglich ist. Hierzu können beispielsweise Hinterschneidungen oder dergleichen vorgesehen sein, so dass die Befestigungselemente nach dem Zusammenfügen der Anschlussteile dauerhaft gesichert sind.

Bei einer bevorzugten Ausführungsform der Tropfkammer ist der Einstechdorn im Zentrum der Befestigungselemente angeordnet und steht über die Befestigungselemente hinaus vor. Dadurch wird erreicht, dass der Einstechdom der Tropfkammer zuerst das Einstechteil des Infusionsbehältnisses durchsticht, bevor die Anschlussteile von Tropfkammer und Infusionsbehältnis miteinander verbunden sind.

Aus fertigungstechnischen Gründen sieht eine weitere bevorzugte Ausführungsform der Tropfkammer vor, dass deren Befestigungsteil als kappenförmiger Körper ausgebildet ist, der auf den Gehäusekörper der Tropfkammer aufgesetzt ist. Der Gehäusekörper der Tropfkammer und das Befestigungsteil können somit in separaten Verfahrensschritten hergestellt werden. Grundsätzlich ist es aber auch möglich, den Gehäusekörper der Tropfkammer und das Befestigungsteil einstückig auszubilden.

Bei einer weiteren besonders bevorzugten Ausführungsform weist der kappenförmige Körper des Befestigungsteils der Tropfkammer ein zylindrisches auf den Gehäusekörper der Tropfkammer aufgesetztes Basisteil auf, an dem die Befestigungselemente angeformt sind.

Das Basisteil des Befestigungsteils der Tropfkammer weist vorzugsweise ein zentrales Aufnahmestück auf, das den Einstechdorn umschließt. Vorzugsweise ist der Einstechdorn einstückiger Bestandteil des Aufnahmestücks, der Einstechdom kann aber auch in das Aufnahmestück eingesetzt und mit demselben verbunden, beispielsweise verschweißt oder verklebt sein..

Das Anschlussteil der Tropfkammer ist aus fertigungstechnischen Gründen vorzugsweise ein Spritzgießteil, das in großen Stückzahlen kostengünstig hergestellt werden kann.

Das erfindungsgemäße Infusionsgerät umfasst die erfindungsgemäße Tropfkammer und eine an einen Patienten anzuschließende Infusionsleitung, die an den proximalen Auslass des Gehäusekörpers der Tropfkammer angeschlossen ist.

Im Folgenden wird die Erfindung unter Bezugnahme auf die Zeichnungen näher erläutert.

Es zeigen:
- Figur 1: ein Infusionsgerät mit Tropfkammer und Infusionsleitung gemäß der Erfindung,
- Figur 2: die Tropfkammer des Infusionsgeräts von Figur 1 in perspektivischer Darstellung,
- Figur 3: einen Infusionsbeutel, an den das erfindungsgemäße Infusionsgerät angeschlossen werden kann,
- Figur 4: einen Schnitt durch den Anschlussteil des Infusionsbeutels zum Entnehmen von Flüssigkeit aus dem Infusionsbeutel von Figur 3 in vergrößerter Darstellung,
- Figur 5: die Verbindung der Tropfkammer des Infusionsgerätes mit dem Anschlussteil des Infusionsbeutels in perspektivischer Darstellung.

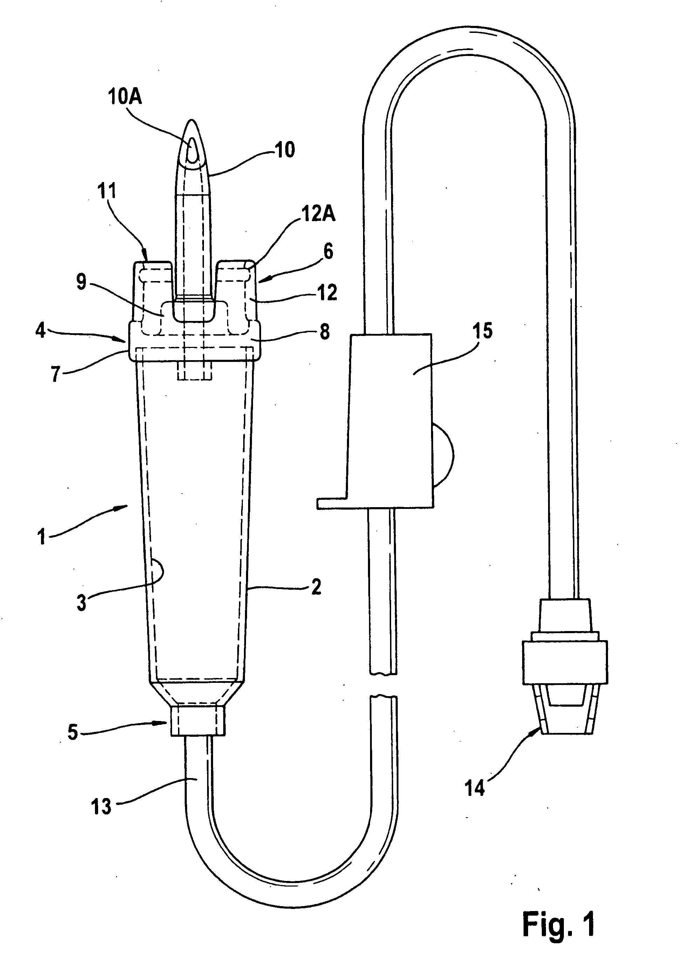

Figur 1 zeigt ein Infusionsgerät gemäß der Erfindung, das über die erfindungsgemäße Tropfkammer verfügt. Nachfolgend wird die erfindungsgemäße Tropfkammer 1 im Einzelnen beschrieben.

Die Tropfkammer 1 weist einen langgestreckten Gehäusekörper 2 aus durchsichtigem Kunststoff auf, der eine Kammer 3 umschließt. Der Gehäusekörper 2 hat einen distalen Einlass 4 und einen proximalen Auslass 5. Die Tropfkammer wird derart angeordnet, dass in der Gebrauchslage der distale Einlass 4 oben und der proximale Auslass 5 unten liegt.

An dem distalen Einlass 4 des Gehäusekörpers 2 ist ein Anschlussteil 6 zum Anschluss der Tropfkammer an ein Infusionsbehältnis vorgesehen. Der Anschlussteil 6 ist als kappenförmiger Körper ausgebildet, der auf den Gehäusekörper 2 aufgesetzt und gegenüber dem Gehäusekörper abgedichtet ist. Der Anschlussteil 6 kann aber auch einstückiger Bestandteil des Gehäusekörpers sein.

Der Anschlussteil 6 der Tropfkammer 1 weist ein auf dem Gehäusekörper 2 sitzenden Basisteil 7 auf, das ein zylindrisches Teilstück 8 hat, das den oberen Rand des Gehäusekörpers 2 umschließt, und ein zentrales Aufnahmestück 9 hat, das einen Einstechdom 10 umschließt. Der Einstechdom 10 weist einen Flüssigkeitskanal 10A auf, der sich in die Kammer 3 des Gehäusekörpers 2 erstreckt. Vorzugsweise ist der Einstechdom 10 einstückiger Bestandteil des Aufnahmestücks 9 des Anschlussteils 6. Zum Belüften des Systems kann noch ein Belüftungskanal vorgesehen sein, in dem ein Sterilfilter angeordnet sein kann.

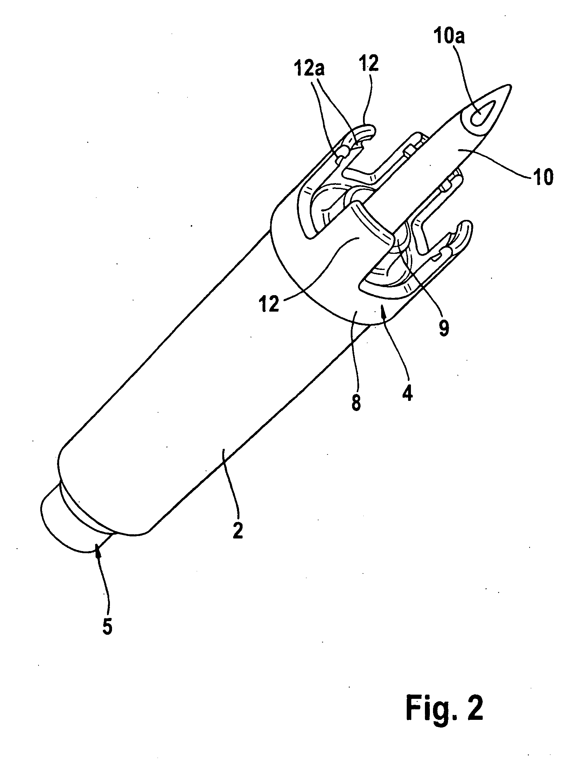

Der Anschlussteil 6 der Tropfkammer 1 verfügt zur lösbaren Befestigung des Anschlussteils 6 der Tropfkammer 1 an einem Anschlussteil eines Infusionsbehältnisses über einen Befestigungsteil 11. Der Befestigungsteil 11 weist eine Mehrzahl, beispielsweise vier entlang des Umfangs eines Kreises angeordnete Befestigungselemente 12 auf, die an das zylindrische Teilstück 7 des Anschlussteils 6 angeformt sind und sich um den Einstechdorn 10 herum nach oben erstrecken. Dabei bilden die Befestigungselemente 12 Teilstücke eines zylindrischen Körpers. Der Einstechdom 20 erstreckt sich über die Befestigungselemente 12 hinaus nach oben (Fig. 2).

Die in gleichem Abstand um den Umfang des zylindrischen Teilstücks 7 angeordneten Befestigungselemente 12 sind derart ausgebildet, dass sie leicht von dem Einstechdom 10 weg nach außen abgespreizt werden können. Hierzu genügt es, dass die Befestigungselemente aus einem Kunststoff gefertigt sind, der leicht nachgibt. Die oberen Randbereiche der Befestigungselemente 12 weisen an den einander zugewandten Innenseiten jeweils eine Nut 12A auf, die entlang des Umfangs eines Kreises verlaufen.

Neben der Tropfkammer 1 umfasst das erfindungsgemäße Infusionsgerät eine Infusionsleitung 13, die an den proximalen Auslass 5 der Tropfkammer angeschlossen ist. An dem proximalen Ende der Schlauchleitung befindet sich ein Anschlussstück 14, beispielsweise ein Luer-Lock-Konnektor zum Anschluss einer Kanüle. An der Infusionsleitung 13 ist zum Unterbrechen der Infusion eine konventionelle Schlauchklemme 15 vorgesehen.

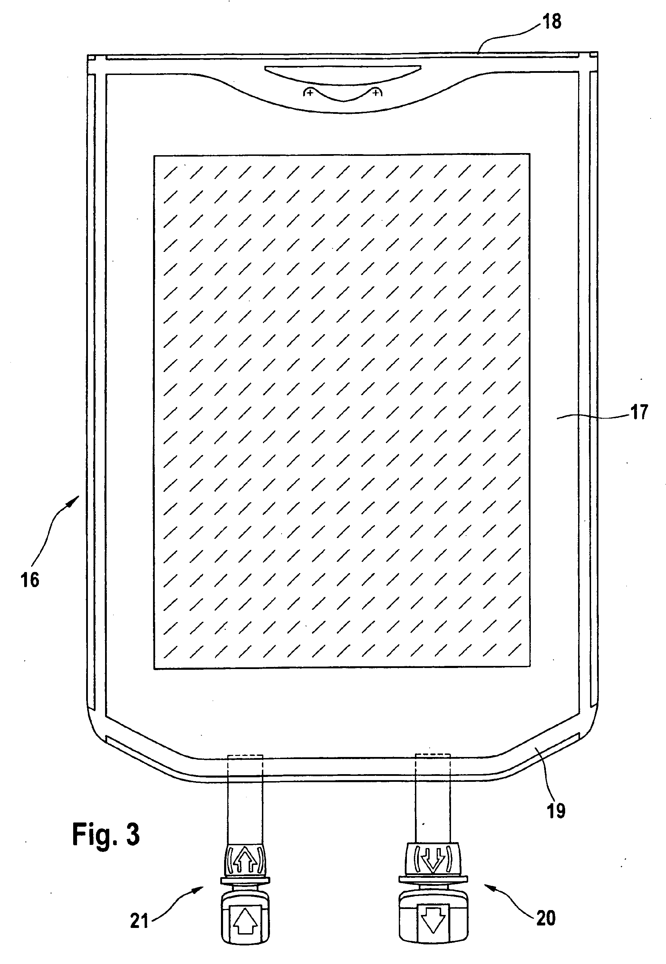

Figur 3 zeigt das Infusionsbehältnis 16, an das die Tropfkammer 1 des Infusionsgerätes angeschlossen werden kann. Das Infusionsbehältnis 16 ist ein herkömmlicher Infusionsbeutel, der aus zwei Folienlagen 17 besteht, die an dem in der Gebrauchslage oberen und unteren Rand 18, 19 miteinander verschweißt sind. In den unteren Rand 19 des Infusionsbeutels sind zwei Anschlussteile 20, 21 eingeschweißt, von denen der eine Anschlussteil 20 einen Entnahmeteil und der andere Anschlussteil 21 einen Zuspritzteil bildet. Der Infusionsbeutel 16 ist mit einer Infusionslösung, beispielsweise mit einem Zytostatika, befüllt.

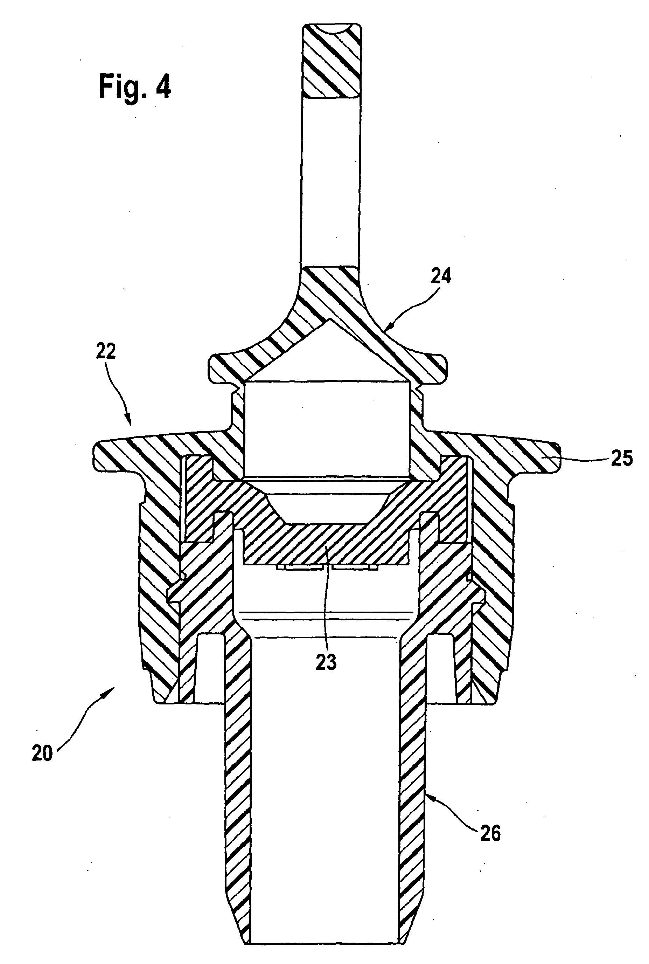

Der Folienbeutel 16 mit Entnahme- und Zuspritzteil 20, 21 ist im Einzelnen in der DE 102 23 560 A1 beschrieben, auf die zum Zwecke der Offenbarung ausdrücklich Bezug genommen wird. Figur 4 zeigt einen Schnitt durch den Entnahmeteil 20 des Folienbeutels 16 in vergrößerter Darstellung. Da der Entnahmeteil 20 in der DE 102 23 560 A1 im Einzelnen beschrieben ist, werden nachfolgend nur die für die Erfindung wesentlichen Komponenten kurz erläutert.

Der Anschlussteil 20 des Infusionsbeutels 16 weist ein beutelseitiges rohrförmiges Teilstück 26 auf, das mit dem Beutel verschweißt ist, und ein anschlussseitiges im wesentlichen zylindrisches Teilstück 22 auf, zwischen denen ein Einstechteil 23, beispielsweise eine selbstabdichtende Membran aus elastischem Material, angeordnet ist. Das anschlussseitige Teilstück 22 des Anschlussteils 20 ist mit einem abdrehbaren Verschlussteil 24 verschlossen. Das anschlussseitige Teilstück 22 des Anschlussteils 20 stellt ein Befestigungsteil zur Befestigung der Tropfkammer dar. Hierzu weist das anschlussseitige Teilstück 22 einen umlaufenden Ansatz 25 auf, der sich radial nach außen erstreckt. Der Querschnitt des umlaufenden Ansatzes 25 entspricht der Form der Nuten 12A der Befestigungselemente 12.

Zum Anschluss der Tropfkammer 1 an den Infusionsbeutel 16 wird der Verschlussteil 24 von dem Anschlussteil 20 abgedreht, so dass der Einstechteil 23 frei liegt. Daraufhin wird die Tropfkammer 1 auf den Befestigungsteil 22 Anschlussteils 20 aufgesetzt, wobei zunächst der Einstechdorn 10 den Einstechteil 23 des Anschlussteils 20 des Infusionsbeutels 16 durchsticht und dann die Befestigungselemente 12 leicht nach außen gebogen werden, bis der umlaufende Ansatz 25 des Anschlussteils 20 einschnappend in den Nuten 12A der Befestigungselemente 12 festgelegt ist. Dadurch wird die Tropfkammer 1 sicher an dem Infusionsbeutel 16 gehalten, ohne dass die Gefahr des Herausrutschens des Anstechdorns 10 aus dem Einstechteil 23 des Anschlussteils 23 besteht.

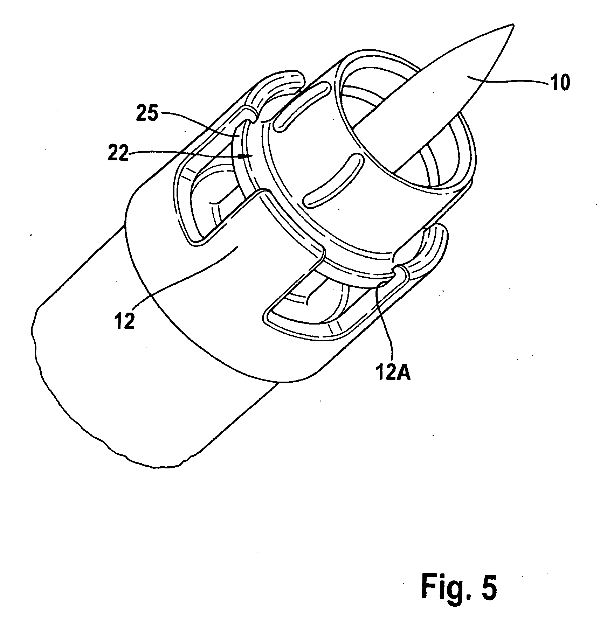

Figur 5 zeigt in perspektivischer Darstellung den Anschlussteil 6 der Tropfkammer 1 und das anschlussseitige Teilstück 22 des Anschlussteils 20 des Infusionsbeutels 16, wobei die selbstabdichtende Membran 23 nicht in das anschlussseitige Teilstück 22 eingesetzt ist. Deutlich ist zu erkennen, wie die Befestigungselemente 12 das anschlussseitige Teilstück 22 des Anschlussteils 20 von allen Seiten umgreifen.

## Patentansprüche

1. Tropfkammer für ein Infusionsgerät mit
einem Gehäusekörper (2), der einen distalen Einlass (4) und einen proximalen Auslass (5) aufweist, und
einem Anschlussteil (6), der an dem distalen Einlass des Gehäusekörpers zum Anschluss der Tropfkammer an einen Anschlussteil eines Infusionsbehältnisses vorgesehen ist, welcher einen in einen Einstechteil des Anschlussteils des Infusionsbehältnisses einstechbaren Einstechdorn (10) mit einem Flüssigkeitskanal (10A) aufweist,
wobei der Anschlussteil (6) der Tropfkammer ein Befestigungsteil (11) zum lösbaren Befestigen des Anschlussteils der Tropfkammer an dem Anschlussteil des Infusionsbehältnisses aufweist,
**dadurch gekennzeichnet, dass**
das Befestigungsteil (11) der Tropfkammer eine Mehrzahl von entlang des Umfangs eines Kreises angeordneten Befestigungselementen (12) aufweist, die an den einander zugewandten Innenseiten Nuten (12A) oder Hinterschneidungen aufweisen, die entlang des Umfangs eines Kreises verlaufen, wobei die Befestigungselemente derart ausgebildet sind, dass die Befestigungselemente aus einer das Befestigungsteil des Infusionsbehältnisses fest umgreifenden Stellung in eine das Befestigungsteil des Infusionsgerätes freigebende Stellung spreizbar sind.

2. Tropfkammer nach Anspruch 1, **dadurch gekennzeichnet, dass** der Einstechdorn (10) im Zentrum der Befestigungselemente (12) über die Befestigungselemente hinaus vorsteht.

3. Tropfkammer nach Anspruch 1 oder 2, **dadurch gekennzeichnet, dass** das Befestigungsteil (11) der Tropfkammer als ein kappenförmiger Körper ausgebildet ist, der auf den Gehäusekörper (2) der Tropfkammer aufgesetzt ist.

4. Tropfkammer nach Anspruch 3, **dadurch gekennzeichnet, dass** der Anschlussteil (6) der Tropfkammer ein zylindrisches auf den Gehäusekörper (2) der Tropfkammer aufgesetztes Basisteil (7) aufweist, an dem die Befestigungselemente (12) angeformt sind.

5. Tropfkammer nach Anspruch 4, **dadurch gekennzeichnet, dass** das Basisteil (8) des Anschlussteils (6) der Tropfkammer ein zentrales Aufnahmestück (9) aufweist, das den Einstechdom (10) umschließt.

6. Tropfkammer nach Anspruch 5, **dadurch gekennzeichnet, dass** der Einstechdom (10) einstückiger Bestandteil des Aufnahmestücks (9) ist.

7. Tropfkammer nach Anspruch 6, **dadurch gekennzeichnet, dass** der Anschlussteil (6) der Tropfkammer ein Spritzgießteil ist.

8. Infusionsgerät mit einer Tropfkammer (1) nach einem der Ansprüche 1 bis 7 und einer an einen Patienten anzuschließenden Infusionsleitung (13), die an den proximalen Auslass (5) des Gehäusekörpers (2) der Tropfkammer angeschlossen ist.

9. Anordnung bestehend aus einem Infusionsbehältnis (16) zur Aufnahme einer medizinischen Flüssigkeit und einem Infusionsgerät nach Anspruch 8, **dadurch gekennzeichnet, dass** das Infusionsbehältnis (16) einen Anschlussteil (20) mit einem Einstechteil (23) zum Einstechen des Einstechdoms (10) der Tropfkammer (1) und ein Befestigungsteil (22) aufweist, wobei das Befestigungsteil (22) des Anschlussteils (20) des Infusionsbehältnisses (16) einen umlaufenden Ansatz (25) aufweist, der derart ausgebildet ist, dass der umlaufende Ansatz in den Nuten (12A) oder Hinterschneidungen des Befestigungsteils (11) der Tropfkammer (1) einschnappend festlegbar ist.

10. Anordnung nach Anspruch 9, **dadurch gekennzeichnet, dass** das Befestigungsteil (22) des Infusionsbehältnisses (16) ein im wesentlichen zylindrisches Teilstück ist, an das der umlaufenden Ansatz (25) angeformt ist.

## Claims

1. A drip chamber for an infusion device with
a housing body (2), which comprises a distal inlet (4) and a proximal outlet (5), and
a connector part (6), which is provided at the distal inlet of the housing body for connecting the drip chamber to a connector part of an infusion container, which comprises a piercing spike (10) with a fluid channel (10A) which can be inserted into a piercing part of the connector part of the infusion container,
wherein the connector part (6) of the drip chamber comprises a securing part (11) for the detachable securing of the connector part of the drip chamber to the connector part of the infusion container,
**characterised in that**
the securing part (11) of the drip chamber comprises a plurality of securing elements (12) disposed along the circumference of a circle, said securing elements comprising grooves (12A) or undercuts at the inner sides facing one another, said grooves or undercuts running along the circumference of a circle, wherein the securing elements are formed in such a way that the securing elements can be splayed from a position firmly engaging around the securing part of the infusion container into a position releasing the securing part of the infusion device.

2. The drip chamber according to claim 1, **characterised in that** the piercing spike (10) in the centre of the securing elements (12) projects beyond the securing elements.

3. The drip chamber according to claim 1 or 2, **characterised in that** the securing part (11) of the drip chamber is constituted as a cap-shaped body which is placed onto the housing body (2) of the drip chamber.

4. The drip chamber according to claim 3, **characterised in that** the connector part (6) of the drip chamber comprises a cylindrical base part (7) which is placed onto the housing body (2) of the drip chamber and on which the securing elements (12) are formed.

5. The drip chamber according to claim 4, **characterised in that** the base part (8) of the connector part (6) of the drip chamber comprises a central mounting piece (9) which surrounds the piercing spike (10).

6. The drip chamber according to claim 5, **characterised in that** the piercing spike (10) is a one-piece component of the mounting piece (9).

7. The drip chamber according to claim 6, **characterised in that** the connector part (6) of the drip chamber is an injection-moulded part.

8. An infusion device with a drip chamber (1) according to any one of claims 1 to 7 and an infusion line (13), which is to be connected to a patient and which is connected to the proximal outlet (5) of the housing body (2) of the drip chamber.

9. An arrangement comprising an infusion container (16) for accommodating a medical fluid and an infusion device according to claim 8, **characterised in that** the infusion container (16) comprises a connector part (20) with a piercing part (23) for the insertion of the piercing spike (10) of the drip chamber (1) and a securing part (22), wherein the securing part (22) of the connector part (20) of the infusion container (16) comprises a peripheral shoulder (25) which is constituted such that the peripheral shoulder can be secured in a snap-in manner in the grooves (12A) or undercuts of the securing part (11) of the drip chamber (1).

10. The arrangement according to claim 9, **characterised in that** the securing part (22) of the infusion container (16) is an essentially cylindrical section on which the peripheral shoulder (25) is formed.

## Revendications

1. Chambre de goutte-à-goutte pour un appareil de perfusion, comportant
un corps de boîtier (2), lequel présente une entrée distale (4) et une sortie proximale (5), et
une pièce de connexion (6), prévue sur l'entrée distale du corps de boîtier pour raccorder la chambre de goutte-à-goutte à une pièce de connexion d'un récipient de perfusion, laquelle comporte un poinçon de perçage (10) pouvant être enfoncé dans une partie à percer de la pièce de connexion du récipient de perfusion, avec un canal de liquide (10A),
la pièce de connexion (6) de la chambre de goutte-à-goutte comportant une pièce de fixation (11) pour la fixation amovible de la pièce de connexion de la chambre de goutte-à-goutte sur la pièce de connexion du récipient de perfusion,
**caractérisée**
**en ce que** la pièce de fixation (11) de la chambre de goutte-à-goutte comporte une pluralité d'éléments de fixation (12) disposés sur la circonférence d'un cercle, lesquels présentent des rainures (12A) sur leurs faces intérieures opposées l'une à l'autre, lesquelles s'étendent sur la circonférence d'un cercle, les éléments de fixation étant réalisés de manière à être écartables d'une position où ils enserrent fermement la pièce de fixation du récipient de perfusion vers une position de déblocage de la pièce de fixation de l'appareil de perfusion.

2. Chambre de goutte-à-goutte selon la revendication 1, **caractérisée en ce que** le poinçon de perçage (10) s'élève au-dessus des éléments de fixation au centre des éléments de fixation (12).

3. Chambre de goutte-à-goutte selon la revendication 1 ou la revendication 2, **caractérisée en ce que** la pièce de fixation (11) de la chambre de goutte-à-goutte est réalisée comme corps en forme de couronne montée sur le corps de boîtier (2) de la chambre de goutte-à-goutte.

4. Chambre de goutte-à-goutte selon la revendication 3, **caractérisée en ce que** la pièce de connexion (6) de la chambre de goutte-à-goutte comporte une pièce de base (7) cylindrique montée sur le corps de boîtier (2) de la chambre de goutte-à-goutte, sur laquelle sont formés les éléments de fixation (12).

5. Chambre de goutte-à-goutte selon la revendication 4, **caractérisée en ce que** la pièce de base (8) de la pièce de connexion (6) de la chambre de goutte-à-goutte comporte une pièce de réception (9) centrale qui entoure le poinçon de perçage (10).

6. Chambre de goutte-à-goutte selon la revendication 5, **caractérisée en ce que** le poinçon de perçage (10) est un composant formé d'un seul tenant de la pièce de réception (9).

7. Chambre de goutte-à-goutte selon la revendication 6, **caractérisée en ce que** la pièce de connexion (6) de la chambre de goutte-à-goutte est une pièce moulée par injection.

8. Appareil de perfusion pourvu d'une chambre de goutte-à-goutte (1) selon l'une des revendications 1 à 7 et d'une conduite de perfusion (13) à brancher sur un patient, laquelle est raccordée à la sortie proximale (5) du corps de boîtier (2) de la chambre de goutte-à-goutte.

9. Dispositif composé d'un récipient de perfusion (16) destiné à recevoir un liquide médical, et d'un appareil de perfusion selon la revendication 8, **caractérisé en ce que** le récipient de perfusion (16) comporte une pièce de connexion (20) avec une partie à percer (23) pour l'enfoncement du poinçon de perçage (10) de la chambre de goutte-à-goutte (1), et une pièce de fixation (22), ladite pièce de fixation (22) de la pièce de connexion (20) du récipient de perfusion (16) présentant un épaulement périphérique (25) réalisé de telle manière que l'épaulement périphérique peut être fixé par enclenchement dans les rainures (12A) de la pièce de fixation (11) de la chambre de goutte-à-goutte (1).

10. Dispositif selon la revendication 9, **caractérisé en ce que** la pièce de fixation (22) du récipient de perfusion (16) est une pièce partielle sensiblement cylindrique, sur laquelle est formé l'épaulement périphérique (25).
